# EUROPEAN PATENT SPECIFICATION

(11) **EP 0 301 203 B1**
(45) Date of publication and mention of the grant of the patent: **07.09.1994**
(21) Application number: 88108844.7
(22) Date of filing: 03.06.1988
(51) Int. Cl.: G01N 33/34, C09D 11/00, G01N 33/36

(54) **Pocket tester for paper and fabrics**
Taschengerät zum Testen von Papier und Stoffen
Appareil de poche pour tester du papier et des tissus

(30) Priority: 27.07.1987 IT 2146587
(43) Date of publication of application: 01.02.1989
(73) Proprietor: FAVINI S.r.l., Rossano Veneto Vicenza (IT)
(72) Inventor: Nicolucci, Clemente, Carmignano di Brenta Padova (IT)

(56) References cited:
- EP-A- 0 017 889
- FR-A- 2 410 565
- FR-A- 2 539 533
- US-A- 3 788 863
- CHEMICAL ABSTRACTS, vol. 73, no. 2, 13 July 1970, Columbus, OH (US); A. KING et al., p. 79, no. 5134p#
- CHEMICAL ABSTRACTS, vol. 101, no. 14, 01 October 1984, Columbus, OH (US); p. 86, no. 112597k#
- PATENT ABSTRACTS OF JAPAN, vol. 10, no. 26 (P-425)[2083], 31 January 1986#

## Description

The present invention relates to a pocket tester for paper and fabrics.

The tester of the invention consists of a common pen or felt-pen containing an ink which comprises at least one solvent and at least a reactive substance which changes of colour when in contact with the particular compound searched in the paper or in the fabric.

The testers of the present invention are very useful because they allow the immediate and easy check of the presence of a particular substance in the paper or in a fabric without any need for an analytical laboratory with the consequent delay in the obtention of the results.

It is known that the paper quality depends on several parameters, from the type of the manufacturing fibers to the reagents used for sizing or coating or for obtaining some particular property in the end product.

Similarly, a fabric is manufactured through processes which employ a lot of chemical products such as e.g. sizing or bleaching or colouring agents.

It is thus very important for a buyer to quickly and surely know if the quality of the purchased paper or fabric corresponds to the one agreed upon.

The pocket testers of the invention allow a check for the presence of aluminium ions or of starch or of a particular colour in the tested product as defined in the claim 1 to be made.

The test is very simple because it consists in tracing a sign on the examined paper or fabric by means of the suitable specific tester for the searched substance and in observing the color change of the traced sign.

The color intensity of the sign often depends on the amount of the searched substance which is present in the paper or in the fabric and this fact allows a quantitative and not only qualitative indication. In such instance the pocket tester is equipped with a small colorimetric reference scale which is directly incorporated on its external surface or in the leaflet of instructions of the tester.

According to a fundamental aspect of the pocket testers of the present invention, the ink usually used in the pens felt-pens is added to or substituted by a solution of a suitable reagent for evidentiating a substance which is possibly present in the paper or in the fabric.

Many reagents can be used, each of them being suitable for a particular substance.

This feature of the invention's pocket testers can be used for identifying one or more substances normally present in the commercial papers or fabrics and also possible substances which are purposely added to the paper or to the fabric in manufacturing stage (tracers).

The use of a particular substance during the manufacturing stage which is identifiable by a pocket tester as per the present invention represents a sure and simple anti-adulteration or anti-falsification method by a simple pen stroke to determine if the paper or the fabric are effectively of the declared origin.

The identification of particular substances present in the so-called safety paper is already known in the art (see FR-A-2 539 533), but said substances must be purposely added to the paper.

In Chemical Abstracts vol. 73, 2, July 13, 1970, 5134p, the use of Archivist's pen is disclosed employing a pH indicator for paper surface testing.

However nobody before the present invention has disclosed or suggested the possibility of testing the quality of paper by identifying its manufacturing process with a simple pocket tester.

According to a fundamental feature of the invention, the reacting substance which is acting as detector of the searched compound is dissolved in a solvent consisting of aliphatic alcohols having from 2 to 6 C atoms; mono- or di- ethyleneglycols, mono- or dipropyleneglycols with one or more hydroxyl groups etherified with aliphatic alcohols having from 1 to 3 C atoms; or mixtures thereof, or aqueous solutions thereof.

The amount of water in the solvent can range between large limits depending on the solubility of the reacting substance in the mixture. Typically the amount of water is comprised between 20% and 80% by wt. on the solvent.

The reacting substances which are dissolved in the solvent have obviously a very different chemical nature because they must react with very different compounds but they have all the common feature of changing of colour or of inducing a change of colour when reacting.

Colouring substances and uv-absorbers are suitable for the exploiting of the invention.

Any other substance or mixture of substances which react by changing colour when in contact with a compound present in the paper or in the fabric is suitable for the present invention.

For example the mixture iodine/potassium iodide which has a blue colour in the presence of starch.

The following examples are useful for better illustrating the invention.

### Example 1

The wick of a felt-pen was saturated with a solution of iodine (1 g) and of potassium iodide (5 g) in a solvent consisting of monomethylether of ethyleneglycol (19 g) in water (75 ml).

The so obtained felt-pen was used as starch indicator and gives a blue-brown colour whose intensity increases the larger the amount of starch present in the paper.

The starch is a sizing agent normally used in paper surface coating to ameliorate the writability and printability of paper. The range of the colour tonality may also indicate the different kind of treatment of the starch.

A uniform colour of the writing indicates a correct distribution of starch on the paper surface. A yellow/colourless sign indicates the absence of starch.

A blue-brown colour indicates the presence of about 1 g/sq. m of starch (which corresponds to a low content of starch in the paper).

A highly intense blue-brown colour indicate the presence of about 5 g/sq. m of starch (corresponding to a paper treated with a large amount of starch).

### Example 2

The wick of a felt-pen was saturated with a solution of fluoroglucine (10 g) in a solvent consisting of 36% hydrochloric acid (30 g), ethyleneglycol monoethylether(25 g) and isopropyl alcohol (10 g).

The so obtained felt-pen was used as a lignified-fibres indicator, i.e. for indicating the wood pulp content (mechanical pulp).

This tester gives a yellow colour on papers or panels consisting of cellulose fibres (chemical pulp) and a red colour on papers consisting of wood mechanical pulp.

The colour intensity and uniformity indicates the percentage content of lignified fibers and by comparison with a reference scale it is possible to evidentiate the lignification grade.

The papers which contain lignified fibers have a lower commercial value because the lignin is a scaling substance which is strongly ageing and is causing yellowing and brittleness.

For example a paper having a high lignified fibers content is used for the printing of national newspapers.

The wood mechanical pulp or wood pulp is very much cheaper than the cellulose pulp.

Many commercial specifications for papers which must be used for art uses or long conservable editions are prescribing the absence of wood pulp.

### Example 3

The wick of a felt-pen was saturated with a solution of panduran blue (10 g) in a solvent consisting of ethyleneglycol monoethylether (20 g) and water (70 g).

The so obtained felt-pen was used as aluminium content indicator (aluminium ions), which is derived from aluminium sulphate or chloride or polychloride or sodium aluminate used as coagulating agent in the manufacturing stage.

The blue colour intensity of the sign depends on the aluminium ions content.

Traditional paper sizing is obtained by precipitating the rosin by aluminium sulphate and of consequence the determination of the presence or absence of alumnium is a useful element for understanding the method which has been used for sizing the paper under examination.

### Example 4

The wick of a felt-pen was saturated with a solution of Cartarex 2L (registered trade mark of Sandoz) (10 g) in a solvent consisting of diethyleneglycol (25 g), ethyleneglycol monoethylether (80 g) and water (120 g).

The so obtained felt-pen was used as a bleaching substances indicator in the tested paper or fabrics.

The sign examined under ultraviolet light (Wood lamp) is not giving the characteristic fluorescence of the possibly present bleaching substance.

## Claims

1. Pocket tester for paper and fabrics consisting of a pen or felt-pen containing an ink consisting of a solution in alcohol or in an water/alcohol mixture of a reacting substance (reagent) for evidentiating a substance (searched substance) which is possibly present in the paper or in the fabrics depending on the type of the raw material used or on particular operations of the manufacturing process, said searched substance being selected from the group consisting of aluminium ions, starch, chemical pulp, mechanical pulp, bleaching agents.

2. Pocket tester as claimed in claim 1. characterised by the fact that said reagent is selected from the group consisting of a mixture of iodine and potassium iodide, a 25% solution of fluoroglucine in concentrated hydrochloric acid, blue of panduran, Cartarex 2L.

3. Pocket tester as claimed in claim 1. characterised by the fact that the alcohol used in said solution is selected from the group consisting of aliphatic alcohols having from 2 to 6 carbon atoms; mono- or di-ethyleneglycols, mono or di-propyleneglycols, having one or more hydroxyl groups etherified with aliphatic alcohols having from 1 to 3 carbon atoms; or mixtures thereof, or their mixtures with from 20% to 80% of water.

4. A method for testing paper quality characterised by checking starch content, wood pulp content and aluminium ions content, by means of a system of pocket testers each one of them consisting of a pen or felt-pen containing a solution of a specific reagent in alcohol or in an water/alcohol mixture, said specific reagent being a mixture of iodine and potassium iodide or a 25% solution of fluoroglucine in concentrated hydrochloric acid or blue of panduran.

## Patentansprüche

1. Taschenprüfgerät für Papier und Gewebe mit einem Schreibstift oder Faserschreiber, der eine Tinte enthält, die aus einer Lösung in Alkohol oder in einer Mischung Wasser/Alkohol einer Reaktionssubstanz (gesuchte Substanz) besteht, die möglicherweise in dem Papier oder Gewebe enthalten ist, abhängig von dem verwendeten Ausgangsstoff oder von besonderen Verfahren bei der Herstellung, wobei die gesuchte Substanz in der Gruppe Aluminiumionen, Stärke, chemische Pulpe, mechanische Pulpe und Bleichmittel enthalten ist.

2. Taschenprüfgerät nach Anspruch 1, dadurch gekennzeichnet, daß das Reagens aus der Gruppe gewählt ist, die eine Mischung von Jod und Kaliumjodid, eine 25%igen Lösung von Fluoroglucin in konzentrierter Salzsäure, Pandurablau und Cartarex 2L enthält.

3. Taschenprüfgerät nach Anspruch 1, dadurch gekennzeichnet, daß der in der Lösung enthaltene Alkohol gewählt ist aus der Gruppe, die aliphatische Alkohole mit 2 bis 6 Kohlenstoffatomen; Mono- oder Di-Ethylenglykole, Mono- oder Di-Propylenglykole mit einer oder mehreren, mit aliphatischen Alkoholen mit 1 bis 3. Kohlenstoffatomen verätherten Hydroxylgruppen; oder deren Mischungen oder deren Mischungen mit 20 % bis 80 % Wasser enthält.

4. Verfahren zum Prüfen der Papierqualität, gekennzeichnet durch die Ermittlung des Gehaltes an Stärke, Holzpulpe und Aluminiumionen mittels eines Systems von Taschenprüfern, von denen jeder aus einem Schreibstift oder Faserschreiber besteht, der eine Lösung eines spezifischen Reagens in Alkohol oder in einer Mischung aus Wasser und Alkohol enthält, wobei das spezifische Reagens eine Mischung von Jod und Kaliumjodid oder eine 25%ige Lösung von Fluorglucin in konzentrierter Salzsäure oder Pandurablau ist.

## Revendications

1. Appareil de contrôle de poche pour le papier et les tissus consistant en un stylographe ou un stylo-feutre contenant une encre consistant en une solution dans de l'alcool ou dans un mélange eau/alcool d'une substance réactive (réactif) afin de mettre en évidence une substance (la substance recherchée) qui peut être présente dans le papier ou dans les tissus en fonction du type de matériau brut utilisé ou lors d'opérations particulières lors du processus de fabrication, ladite substance recherchée étant choisie parmis le groupe consistant en ions aluminium, amidon, pulpe chimique, pulpe mécanique, agents de blanchiments.

2. Appareil de contrôle de poche selon la revendication 1, caractérisé en ce que le réactif est choisi dans le groupe sonsistant en un mélange d'iode ou d'iodure de potassium, une solution à 25% de fluoroglucine dans de l'acide chlorhydrique concentré, du bleu de panduran, du Cartarex 2L.

3. Appareil de contrôle de poche selon la revendication 1, caractérisé en ce que l'alcool utilisé dans la solution est choisi dans le groupe consistant en alcools aliphatiques ayant de 2 à 6 atomes de carbone; en mono- ou diéthylèneglycols, ayant un ou plusieurs groupes estérifiés avec des alcools aliphatiques ayant de 1 à 3 atomes de carbone; ou dans des mélanges de ceux-ci, ou dans des mélanges ayant de 20 à 80% d'eau.

4. Méthode pour tester la qualité du papier caractérisée en ce qu'elle contrôle le contenu en amidon, en pulpe de bois et en ions aluminium, au moyen d'un système d'appareils de contrôle de poche, chacun de ceux-ci consistant en un stylographe ou un stylo-feutre contenant une solution d'un réactif spécifique dans de l'alcool ou dans un mélange eau/alcool, ledit réactif spécifique étant un mélange d'iode et d'iodure de potassium ou une solution à 25% de fluoroglucine dans de l'acide chlorhydrique concentré ou du bleu de panduran.
